(19) Europäisches Patentamt
European Patent Office
Office européen des brevets

(11) Publication number: **0 434 345 A1**

# (12) EUROPEAN PATENT APPLICATION

(21) Application number: **90313790.9**

(51) Int. Cl.[5]: **C07D 257/02**, A61K 49/04

(22) Date of filing: **17.12.90**

(30) Priority: **22.12.89 US 454883**

(43) Date of publication of application:
**26.06.91 Bulletin 91/26**

(84) Designated Contracting States:
**AT BE CH DE DK ES FR GB GR IT LI LU NL SE**

(71) Applicant: **E.R. SQUIBB & SONS, INC.**
**Lawrenceville-Princeton Road**
**Princeton New Jersey 08543-4000(US)**

(72) Inventor: **Dischino, Douglas D.**
**27 Sweetgum Lane**
**Monmouth Junction, New Jersey(US)**

(74) Representative: **Thomas, Roger Tamlyn et al**
**D. Young & Co. 10 Staple Inn**
**London WC1V 7RD(GB)**

(54) **10-(2'-hydroxy-3'-alkoxy)-1,4,7-triscarboxymethyl-1,4,7,10-tetraazacyclododecanes.**

(57) Metal chelating ligands useful as contrast agents are disclosed having the formula

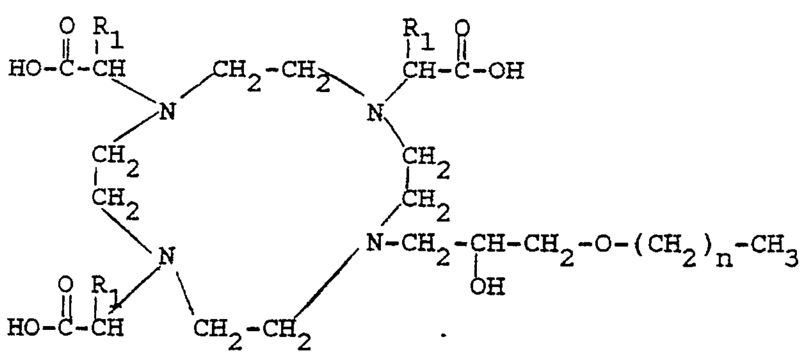


**EP 0 434 345 A1**

# 10-(2'-HYDROXY-3'-ALKOXY)-1,4,7-TRISCARBOXYMETHYL-1,4,7,10-TETRAAZACYCLODODECANES

Metal-chelating ligands are useful in diagnostic medicine as contrast agents. X-ray imaging, radionuclide imaging, ultrasound imaging and magnetic resonance imaging can each be enhanced by the use of a metal atom bound to a chelating ligand. For example, a chelating ligand can become a radiopharmaceutical when it is prepared as a chelate complex with $^{99m}Tc$, $^{111}In$, $^{67}Ga$, $^{140}La$, $^{169}Yb$, $^{68}Ga$, $^{90}Y$, $^{188}Re$, $^{153}Sm$ or other radioactive metal ions. When a chelating ligand is complexed with the stable isotopes of the lanthanides, tantalum, bismuth or other elements with molecular weight higher than iodine, the resulting complex absorbs x-rays sufficiently to act as an x-ray contrast agent. In some cases, the agents that are useful in x-ray imaging absorb, reflect or scatter ultrasound radiation sufficiently to be used as an ultrasound agent. If a chelating ligand is complexed with a paramagnetic metal atom that has a symmetric electronic ground state (e.g., $Gd^{+3}$, octahedral ($Mn^{+2}$ or $Fe^{+3}$, $Cr^{+3}$) the resulting complex will be useful as a spin relaxation catalyst that is used in magnetic resonance imaging (also known as NMR imaging) as a contrast agent. If a chelating agent is complexed with a paramagnetic metal atom that has an unsymmetrical electronic ground state (e.g., dysprosium(III), holmium(III) and erbium(III)), the resulting complex will be useful as a chemical shift agent in magnetic resonance imaging or in magnetic resonance in vivo spectroscopy.

Desirable physical properties of the chelator differ depending on the diagnostic or therapeutic purpose of the metal chelate. Desirable physical properties common to all uses are high thermodynamic affinity for the metal ion bound to the chelator, high selectivity for the metal ion of interest, high kinetic inertia to dissociation of the metal ion from the metal chelate, and ease of synthesis. when it is desired to use the metal chelate as a contrast medium for NMR imaging or general purpose X-ray imaging, the desirable physical properties are high water solubility, and viscosity and osmolality of a formulated drug as close as possible to those of human blood.

Human blood has an osmolality of 0.3 Osmol/ kg-water. Hyperosmolality is a well known contributor to adverse patient reactions to injected contrast media, and the lower osmolality of newer x-ray agents is due to their being nonionic molecules (possessing a net zero overall charge) (Shehadi, WH; "Contrast media adverse reactions: occurrence, reoccurrence and distribution patterns" Radiol. 1982, 143, 11 - 17. Bettman, MA; "Angiographic contrast agents; conventional and new media compared", Am. J. Roetgen. 1982, 139, 787 - 794. Bettman, MA and Morris Tw; Recent advances in contrast agents, Radiol. Clin. North Am. 1986, 24, 347 - 357.). Gadolinium-based NMR agents in the prior art that are useful have a net negative overall charge; their aqueous formulated solutions have high osmolality. For example, $Gd(DTPA)^{2-}$ where DTPA stands for diethylenetriaminepentaacetic acid is formulated for use at 0.5 M in water as the N-methylglucamine salt. The osmolality of the solution is 1.6 to 2.0 Osmol/kg-water. The preferred new gadolinium complexes of the present invention are nonionic - they are not salts. when these non-ionic gadolinium complexes are formulated at 0.5 M in water the osmolality of the solutions is 0.3 -0.6 Osmol/kg-water. The complex should be generally inert to interaction with the body other than general tissue distribution and excretion, usually by the renal route. These properties are also important to NMR imaging, but, in addition, the effectiveness of an agent for NMR imaging can be increased by altering the chemical structure so as to increase the ability of the metal chelate to affect the relaxation times of water protons.

In radiopharmaceutical imaging the doses administered are relatively small so that matching the drug formulation's physical properties to those of human blood is relatively unimportant. In this use biological specificity is more important. In particular, one could use $^{99m}Tc$ as the metal and a chelating ligand which is functionalized with a biologically active entity such as a bile acid, fatty acid, amino acid, peptide, protein, or one of numerous chemical entities known to bind receptors in vivo. NMR contrast media may also make use of biological specificity.

In radiopharmaceutical therapy, the metal ions may be chosen from among those known in the art; for example, $^{90}Y$, $^{188}Re$, $^{153}Sm$. For this purpose the chelating ligand is generally covalently bound to a disease specific entity such as a monoclonal antibody. When the metal-chelator-antibody conjugate is injected into humans, it concentrates at the disease site, usually a malignant tumor. In this use the chelating ligand must contain a reactive functionality which allows for a covalent bond to be formed between the chelating ligand and the antibody. Important characteristics of the reactive functionality are as follows: 1) It must be covalently attached to the chelator such that it does not significantly diminish the affinity of the chelator for the metal ion. 2) It must allow simple synthesis in high yield of metal-chelator-antibody conjugates. The conjugate so-formed should have maximal affinity for its antigen, such affinity being minimally diminished as a result of covalently attaching the metal-chelator. 3) It should ideally allow for rapid excretion and/or optimal dosimetry of the radioactive metal chelator in the event that the metal-chelator-antibody conjugate is decomposed or metabolized in vivo.

When the metal is non-radioactive and paramagnetic such as gadolinium (III) the bifunctional chelate is useful in magnetic resonance imaging as a contrast agent, either as a discrete molecule or bound to substances such as lipids, sugars, alcohols, bile acids, fatty acids, receptor-binding ligands, amino acids, peptides, polypeptides, proteins, and monoclonal antibodies. When the metal is radioactive, such a yttrium-(III) as $^{90}Y$, the bifunctional chelate is useful in labeling monoclonal antibodies for use in radiotherapy. When the metal is $^{99m}Tc$, $^{111}In$, $^{201}Tl$ $^{67}Ga$, $^{68}Ga$ or the like, the chelate is useful in radiopharmaceutical imaging.

Two general methods have been employed for making bifunctional chelates from chelating agents. In the first method one or more carboxylic acid groups of a polyamino, polycarboxylic acid chelator are activated by conversion into such activated groups as internal or mixed anhydrides, activated esters (e.g. p nitro phenyl, N-hydroxysuccinimide, etc.) or with other derivatives known to those skilled in the art. The activated acid group is then reacted with the protein. The metal ion is then added to the protein-chelator complex.

There are two problems with this method. First, using a potential donor group, the carboxylic acid, to react with the protein can diminish the strength of the chelate and contribute to the chemical lability of the metal ion. The second problem arises because the chelating ligands have several carboxylates that are not uniquely reactive. When the chelating ligand is combined with an activating agent more than one species can result because the number and chemical position of the groups activated cannot be adequately controlled. When a mixture of such variously activated chelating ligands is added to protein, protein-chelator complexes of variable and uncertain chelating strength can be formed. Also, multiple activation of carboxylic acids on a chelator leads to intra-and inter-molecular crosslinking which is a major source of decreased immunospecificity. This problem could be overcome by separating all of the products formed from the reaction of the activating agent with the chelating ligand, but that process is very laborious and makes the overall synthesis highly inefficient.

The second method for making a bifunctional chelate is to prepare a chelating ligand with a unique reactive function, such as an isothiocyanate, attached to the chelating ligand at a position that does not substantially diminish the strength with which the chelating ligand binds the metal ion. An article entitled "Synthesis of 1-(p-isothiocyanatobenzyl) derivatives of DTPA and EDTA. Antibody Labeling and Tumor-Imaging Studies" by Martin W. Brechbiel, Otto A. Gansow, Robert W. Atcher, Jeffrey Schlom, Jose Esteban, Diane E. Simpson, David Colcher, Inorganic Chemistry, 1986, 25, 2772 is illustrative of the above second method.

Using the present invention, it is possible to provide metal-chelating ligands whose metal chelate complexes have low osmolality and low acute toxicity, and which, when complexed with a paramagnetic metal atom, are effective as relaxation catalysts in magnetic resonance imaging, and also bifunctional metal-chelating ligands that have the ability to covalently bind to proteins or other biologically active molecules thereby imparting biological specificity to the metal chelate complex, and that are thermodynamically stable, kinetically inert and, when desired, electrically neutral.

The present invention provides compounds having the formula

I

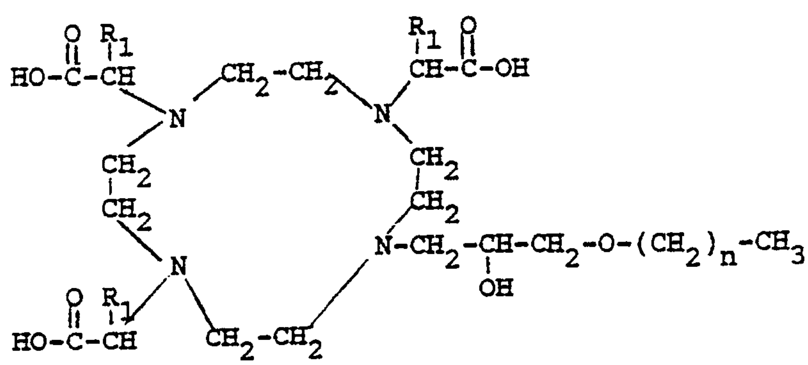

wherein n is zero or an integer from one to five. The preferred values of n are zero, one, two and three.

$R_1$ is hydrogen or alkyl

The term "alkyl" refers to both straight and branched chain groups. Those groups having 1 to 5 carbon atoms are preferred and methyl is the most preferred alkyl group.

The term $(CH_2)_n$ includes straight or branched chain radicals having from 0 to 18 carbons in the straight

chain and may contain one or more lower alkyl substituents. Examples of $(CH_2)_n$ groups include

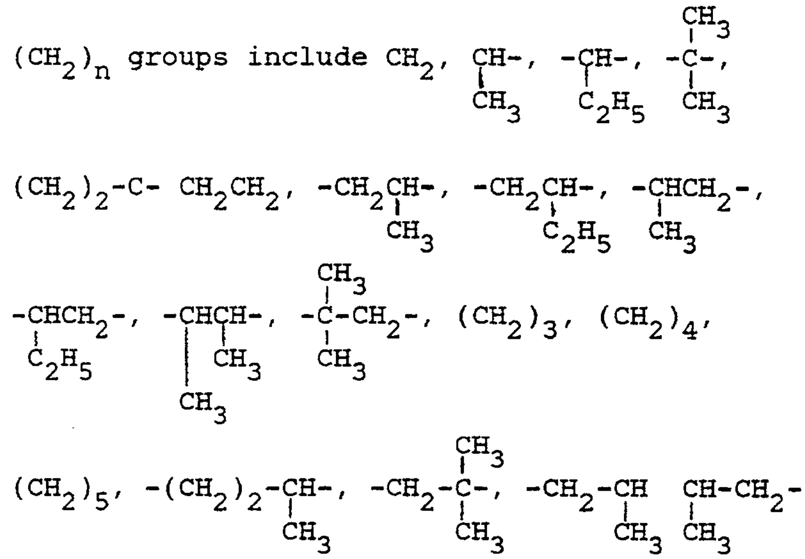

The compounds of formula I, and salts thereof, can be complexed with a paramagnetic metal atom and used as relaxation enhancement agents for magnetic resonance imaging. These agents, when administered to a mammalian host (e.g., humans) distribute in various concentrations to different tissues, and catalyze relaxation of protons (in the tissues) that have been excited by the absorption of radio-frequency energy from a magnetic resonance imager. This acceleration of the rate of relaxation of the excited protons provides for an image of different contrast when the host is scanned with a magnetic resonance imager. The magnetic resonance imager is used to record images at various times generally before and after administration of the agents, and the differences in the images created by the agents' presence in tissues are used in diagnosis. In proton magnetic resonance imaging, paramagnetic metal atoms such as gadolinium(III), and octahedral manganese(II), chromium(III), and iron(III) (all are paramagnetic metal atoms with a symmetrical electronic configuration) are preferred as metals complexed by the ligands of formula I; gadolinium (III) is most preferred due to the fact that it has the highest paramagnetism, low toxicity, and high lability of coordinated water.

The metal-chelating ligands of formula I can be complexed with a lanthanide (atomic number 58 to 71) and used as chemical shift agents in magnetic resonance imaging or in magnetic resonance in vivo spectroscopy.

While the above-described uses for the metal-chelating ligands of formula I are preferred, those working in the diagnostic arts will appreciate that the ligands can also be complexed with the appropriate metals and used as contrast agents in x-ray imaging, radionuclide imaging and ultrasound imaging.

Use In Imaging

To use the ligands of this invention for imaging, they must first be complexed with the appropriate metal. This can be accomplished by methodology known in the art. For example, the metal can be added to water in the form of an oxide or in the form of a halide and treated with an equimolar amount of a ligand of formula I. The ligand can be added as an aqueous solution or suspension. Dilute acid or base can be added (if needed) to maintain a neutral pH. Heating at temperatures as high as 100 °C for periods up to four hours is sometimes required, depending on the metal and the chelator, and their concentrations.

Pharmaceutically acceptable salts of the metal complexes of the ligands of this invention are also useful as imaging agents. They can be prepared by using a base (e.g., an alkali metal hydroxide, meglumine or arginine) to neutralize the above-prepared metal complexes while they are still in solution. Some of the metal complexes are formally uncharged and do not need cations as counterions. Such neutral complexes are preferred as intravenously administered x-ray and NMR imaging agents over charged complexes because they provide solutions of greater physiologic tolerance due to their lower osmolality.

Sterile aqueous solutions of the chelatecomplexes can be administered to mammals (e.g., humans) orally, intrathecally and especially intravenously in concentrations of 0.003 to 1.0 molar. For example, for

the visualization of brain lesions in canines using magnetic resonance imaging, a gadolinium complex of a ligand of formula I can be administered intravenously at a dose of 0.05 to 0.5 millimoles of the complex per kilogram of animal body weight, preferably at a dose of 0.1 to 0.25 millimole/kilogram. For visualization of the kidneys, the dose is preferably 0.05 to 0.25 millimoles/kilogram. For visualization of the heart, the dose is preferably 0.25 to 1.0 millimoles/kilogram. The pH of the formulation will be between about 6.0 and 8.0, preferably between about 6.5 and 7.5. Physiologically acceptable buffers (e.g., tris(hydroxymethyl)-aminomethane) and other physiologically acceptable additives (e.g., stabilizers such as parabens) can be present.

The compounds of formula I can be prepared by reacting a compound having the formula

II

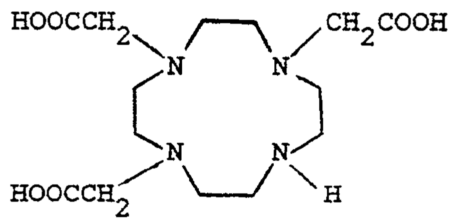

II

with a compound having the formula

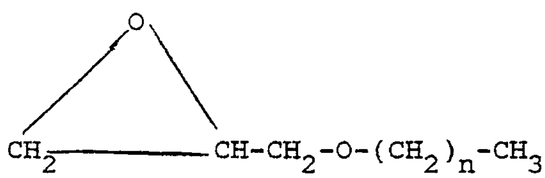

in the presence of NaOH and water. The resulting ligand is reacted with a metal oxide such as $Gd_2O_3$ in water to form the corresponding gadolinium complex.

The following examples are specific embodiments of this invention.

Example 1

1,4,7-Tris(carboxymethyl)-10-(2'-hydroxy-3'-methoxypropyl)-1,4,7,10-tetraazacyclododecanatogadolinium a) 1,4,7-Tris(carboxymethyl)-10-(2'-hydroxy-3'-methoxypropyl)-1,4,7,10-tetraazacyclododecane

Into a 50 ml round bottom flask containing 30 ml of $H_2O$ was added 2.4 g. (0.060 mol) of NaOH. The solution was cooled in an ice-bath and 5.19 g. (0.0144 mol, corrected for $H_2O$) of 1,4,7-Tris-(carboxymethyl)-1,4,7,10-tetraazacyclododecane was added. The pH of the solution was 11.7. To the cooled solution (appr. 10 °C) was added 2.64 g. (0.030 mol) of glycidyl methyl ether. The reaction was allowed to stir at room temperature in a closed vessel for 48 hours. HPLC analysis of the reaction mixture after 48 hours indicated complete conversion. The reaction solution was then diluted to 0.25 L with $H_2O$ and applied to a 5 x 45 cm column of Bio-Rad AG 1-X8 strong anion exchange resin (formate form). The flow rate of the column was approximately 10 - 15 ml/min. The eluant was monitored by means of a conductivity detector. After the conductivity detector indicated that no salts were eluting (appr. 10 L), the column was rinsed with 2 L of 0.5 M $HCO_2H$. The eluate was collected and concentrated on a rotary evaporator. The solid was dissolved in 200 ml of anhydrous methanol and concentrated on a rotary evaporator. The solid was then dissolved in 0.5 L of $H_2O$ and applied to a 1L column of PVP resin. The flow rate of the column was approximately 10 - 15 ml/min. The column was eluted with $H_2O$ and the eluate monitored by a conductivity detector. The first 2 L of the eluate were collected. The solution was concentrated on a rotary evaporator. The solid was then dissolved in 200 ml of methanol and concentrated on a rotary evaporator. The solid was then dried in a vacuum oven at 60 °C for 14 hours to yield 6.1 g. (88.3% allowing for 9.25% $H_2O$) of 1,4,7-tris(carboxymethyl)-10-(2'-hydroxy-3'-methoxypropyl )-1,4,7, 10-tetraazacyclododecane.
Anal. Calcd. (found) for $C_{18}H_{34}N_4O_8 \cdot 2.46\ H_2O$: C, 45.15(45.03); H, 8.19(8.01); N, 11.70 (11.55); $H_20$, (9.25 by KF dissolution)

b) 1,4,7-Tris(carboxymethyl)-10-(2'-hydroxy-3'-methoxypropyl )-1,4,7,10-tetraazacyclododecanatogadolinium

Into a 100 ml round bottom flask was dissolved 4.34 g. (0.0091 mol corrected for $H_2O$) of 1,4,7-tris-(carboxymethyl )-10-(2'-hydroxy-3'-methoxypropyl)-1,4,7,10-tetraazacyclododecane in 20 ml of $H_2O$ and 1.99 g. (0.0055 mol) of $Gd_2O_3$. The reaction mixture was allowed to stir at 90°C for 20 hours. After 20 hours the solution was filtered through a 0.2 micron filter and the solution concentrated on a rotary evaporator. HPLC analysis of the solid indicated approximately 10% free $Gd^{+3}$. The solid was dissolved in 500 ml. of $H_2O$ and applied to a 1 L column of Chelex-100 ($NH_4^+$ form). The flow rate of the column was approximately 10-13 ml/min. The eluate was monitored by a conductivity detector. The eluate was collected and concentrated on a rotary evaporator. The solid was dissolved in 20 ml of $H_2O$ and purified via preparative HPLC. The desired peak was collected and the solution concentrated on a rotary evaporator to yield 4.5 g. (78.2% corrected for $H_2O$) of 1,4,7-tris(carboxymethyl)-10-(2'-hydroxy-3-methoxypropyl )-1,4,7,10-tetraazacyclododecanatogadolinium.
Anal. Calcd. (found) for $C_{18}H_3N_4O_{8Gd} \cdot 0.86\ H_2O$: C, 35.78(35.72); H, 5.46 (5.71); N, 9.28 (9.23); $H_2O$, (2.56 by KF dissolution)

## Claims

1. A compound of the formula

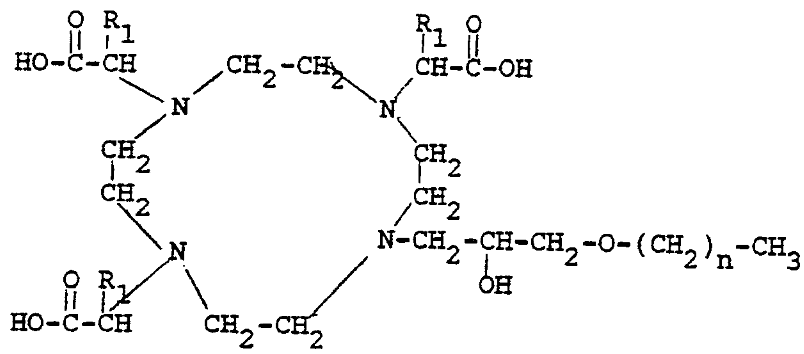

wherein $R_1$ is hydrogen or alkyl and n is zero or an integer from one to five.

2. A compound according to Claim 1 wherein n is zero, one, two or three.

3. A compound according to the Claim 1, 1,4,7-Tris(carboxymethyl)-10-(2'-hydroxy-3'-methoxypropyl )-1,4,7,10-tetraazacyclododecane.

4. A complex, or a salt of a complex, of a metal atom and a metal chelating ligand having the formula

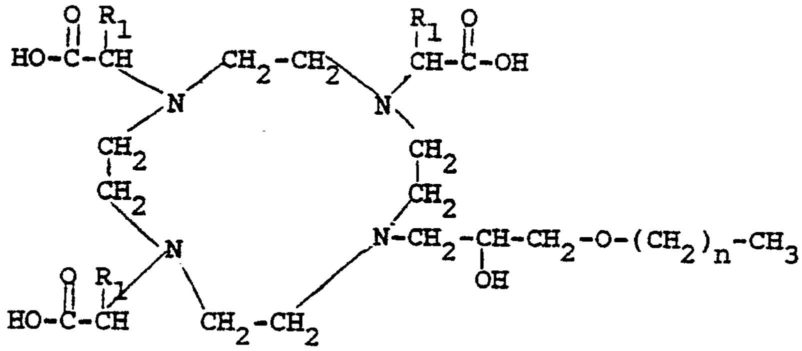

wherein $R_1$ is hydrogen or alkyl;
and n is zero or an integer from one to five.

**5.** A complex according to Claim 4 where the metal atom is gadolinium(III).

**6.** A complex in accordance with Claim 4, 1.4.7-Tris(carboxymethyl)-10-(2'-hydroxy-3'-methoxypropyl )-1,4,7,10-tetraazacyclododecanatogadolinium.

**7.** A process for preparing compounds

I

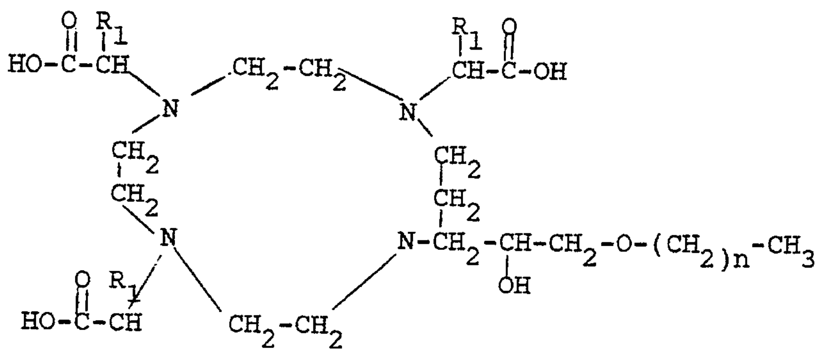

wherein n is zero or an integer from one to five and $R_1$ is hydrogen or alkyl by reacting a compound of the formula

II

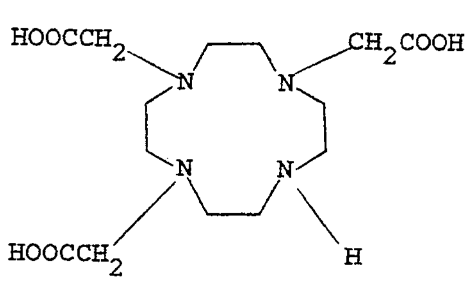

with a compound of formula

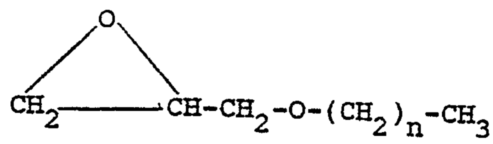

in the presence of strong base and water.

**8.** A process according to Claim 7 wherein the resulting ligand is reacted with a metal oxide in water to form the corresponding metal complex.

**9.** A process according to Claim 8 wherein the metal oxide is $Gd_2O_3$.

**10.** A complex according to any one of Claims 4-6 for use as a diagnostic contrast agent.

European Patent Office

# EUROPEAN SEARCH REPORT

Application Number

EP 90313790.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DE - A1 - 3 625 417 (SCHERING) * Claims 1,6,8; example 6 * -- | 1,4,5, 6,10 | C 07 D 257/02 A 61 K 49/04 |
| A | EP - A2 - 0 292 689 (SQUIBB) * Claims 1,62 * -- | 1,4,5, 6,10 | |
| A | EP - A1 - 0 232 751 (SQUIBB) * Claims 1,17 * -- | 1,4,5, 6,10 | |
| A | EP - A1 - 0 287 465 (GUERBET) * Claims 1,16 * -- | 1,4,5, 6,10 | |
| A | EP - A1 - 0 326 226 (NYCOMED) * Claims 1,23 * -- | 1,10 | |
| A | EP - A1 - 0 299 795 (NYCOMED) * Claims 7,12 * -- | 1,15 | TECHNICAL FIELDS SEARCHED (Int. Cl.5) |
| | CHEMICAL ABSTRACTS, vol. 85, no. 25, December 20, 1976, Columbus, Ohio, USA STETTER, HERMANN et al. "Complexing with tetraaza-cycloalkane-N,N',N'',N'''-tetraacetic acids in relation to ring size" page 399, column 1, abstract-No. 198 997b & Angew. Chem. 1976, 88(22), 760 ---- | | C 07 D 257/00 |

The present search report has been drawn up for all claims

| Place of search | Date of completion of the search | Examiner |
|---|---|---|
| VIENNA | 01-03-1991 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)